# EUROPEAN PATENT APPLICATION

(11) **EP 3 069 654 A1**
(43) Date of publication of application: **21.09.2016**
(21) Application number: 15160031.9
(22) Date of filing: 20.03.2015
(51) Int. Cl.: A61B 5/00, A61B 18/14

(54) **Ablation catheter**

(71) Applicant: Otto-von-Guericke-University, 39106 Magdeburg (DE)
(72) Inventor: Friebe, Michael, 45657 Recklinghausen (DE); Axel, Boese, 39106 Magdeburg (DE)

(57) **Abstract**

The present invention relates to catheters and devices designed to ablate tissue.

## Description

### FIELD OF THE INVENTION

The present invention relates to catheters and devices designed to ablate tissue.

### BACKGROUND ART

The American Cancer Society estimates that approximately one million cases of cancer are diagnosed in the United States each year. Seventy-seven percent of cancers are diagnosed in men and women over the age of 55, although cancer may affect individuals of any age. Surgical removal is one of four main ways that tumors are treated. Chemotherapy, radiation therapy, and biological therapy are other treatment options. Surgery may be used to remove tumors for diagnostic or therapeutic purposes. For diagnostic purposes, a small piece of tissue for diagnostic testing is obtained, a procedure called biopsy, and the sample of the abnormal tissue is examined histologically. There are several known biopsy techniques used to diagnose cancer. These include: fine needle aspiration biopsy (FNAB), core needle biopsy (CNB), stereotactic core needle biopsy, vacuum-assisted core biopsy, MRI guided biopsy, and surgical biopsy.

Once surgical removal has been decided, a surgical oncologist will remove the entire tumor, taking with it a large section of the surrounding normal tissue. The healthy tissue is removed to minimize the risk that abnormal tissue is left behind. Typically the surgeon uses a scalpel to cut a one-piece specimen and the surrounding tissue. The margin of the removed specimen will be examined to determine whether the cancer has been completely removed. If there are cancer cells close to the edge of the specimen, re-excision or second invasive surgical procedure to remove more tissue is performed. To avoid multiple excision and repeated invasive surgeries, the surgeon makes a large incision to provide access for scalpel to excise the lesion in order to obtain a larger than needed one-piece specimen. This results in scarring and excessive amount of tissue being removed. Further, repeated invasive surgeries may lead to patient complications. Therefore, it would be desirable to develop a device and catheter that is minimally invasive and can solve the above-mentioned problems.

### DISCLOSURE OF THE INVENTION

### SUMMARY OF THE INVENTION

The present invention provides a catheter being designed to ablate a biological tissue. Said catheter comprising (a) an elongate having a lumen and a surface extending from a proximal end to a distal end, wherein said surface of the elongate comprising a cavity at the distal end; and (b) an sliding element disposed on the surface of the elongate being designed to slide in longitudinal direction of the elongate and to rotate relative to the elongate, wherein the sliding element comprising a plurality of cuts at the distal end of the elongate and being designed to expand radially from the axis of the sliding element when said sliding element slides in the distal direction of the elongate, thereby ablating the biological tissue.

In Further embodiment, the catheter according to the preceding embodiment comprising a trocar disposed on the sliding element.

In Further embodiment, the elongate of the catheter according to any of the preceding embodiments being designed to connect to a vacuum source, said vacuum source being designed to create a vacuum within the lumen of the elongate, thereby extracting the ablated biological tissue through the cavity.

In further embodiment, the present invention provides the catheter according to any of the preceding embodiments, wherein the cut being designed to comprise a cauterizing element.

In further embodiment, the present invention provides the catheter according to any of the preceding embodiments, wherein the cauterizing element being designed to be electrically conductive.

In further embodiment, the cauterizing element of the catheter according to any of the preceding embodiments being designed to release cauterizing chemicals.

In further embodiment, the cut as part of structure of the catheter according to any of the preceding embodiments being designed to comprise a coating containing a pharmaceutical agent.

In another embodiment, the present invention provides a catheter designed to ablate a biological tissue from a subject comprising (a) an elongate having a proximal end and a distal end, (b) a first sliding element located at the distal end of the elongate being designed to slide in longitudinal direction of the elongate; (c) a second sliding element located at the proximal end of the elongate being designed to slide in longitudinal direction of the elongate; and (d) a plurality of flexible ablation elements being designed to connect to the first and the second sliding elements and the ablation elements being designed to expand radially from the axis of the elongate when the first and/or the second sliding element slide in longitudinal direction of the elongate.

In further embodiment, the elongate of the present invention comprising stopping elements to prevent motion of the first and/or the second sliding element in longitudinal direction of the elongate.

In further embodiment, the first and/or the second sliding element of the present invention being designed to connect to at least one controller, wherein the at least one controller being designed to push and/or pull the first and/or the second sliding element in longitudinal direction of the elongate.

In further embodiment, the first and/or the second sliding element of the present invention being designed to comprise a locking system to prevent motion of the first and/or the second sliding element.

In further embodiment, the ablation element of the present invention being designed to comprise a cauterizing element.

In further embodiment, the cauterizing element of the present invention being designed to be electrically conductive.

In further embodiment, the cauterizing element of the present invention being designed to release cauterizing chemicals.

In further embodiment, the ablation element of the present invention being designed to comprise a coating containing a pharmaceutical agent.

In further embodiment, the catheter of present invention further comprising at least one controller being designed to connect to the first and/or the second sliding element and being designed to pull and/or push the first and/or the second sliding element in longitudinal direction of the elongate.

The present invention further provides a device designed to ablate a biological tissue from a subject. Said device comprising (a) a housing having a lumen; (b) a catheter comprising (i) an elongate having a proximal end and a distal end, (ii) a first sliding element located at the distal end of the elongate being designed to slide in longitudinal direction of the elongate; (iii) a second sliding element located at the proximal end of the elongate being designed to slide in longitudinal direction of the elongate; (iv) a plurality of flexible ablation elements being designed to connect to the first and the second sliding elements and the ablation elements being designed to expand radially from the axis of the elongate when the first and/or the second sliding element slide in longitudinal direction of the elongate; wherein the catheter is disposed within the lumen and said elongate is both movable and rotatable relative to the hosing, thereby moving and rotating the sliding and ablation elements; and (c) at least one controller being designed to move the first and/or the second sliding element in longitudinal direction of the elongate and to rotate the elongate, thereby ablating the biological tissue.

In further embodiment, the device of the present invention further comprising a vacuum source designed to create a vacuum within the housing, thereby extracting the ablated biological tissue.

In further embodiment, the ablation element of the device according to the present invention further being designed to comprise a cauterizing element.

In further embodiment, the device of the present invention comprising an electrical source coupled to the cauterizing elements.

In further embodiment, the cauterizing element of the device according to the present invention being designed to release a cauterizing chemical.

In further embodiment, the ablation element of the device according to the present invention being designed to comprise a coating containing a pharmaceutical agent.

In further embodiment, the catheters and the devices of the present invention according to any of the preceding embodiments being designed to be monitored via Real-time imaging to assist in placement of the catheter and device and in ablating and removing the biological tissue.

Additional embodiments provide methods of ablating a biological tissue from a subject.

### DRAWINGS

Those of skill in the art will understand that the drawings, described below, are for illustrative purposes only. The drawings are not intended to limit the scope of the present teachings in any way.
FIG. 1 illustrates the catheter according to the present invention with the cuts (16) as part of structure of the sliding element (2) at their stretched position.
FIG. 2 illustrates the catheter according to the present invention with the cuts (16) as part of structure of the sliding element (2) expanded radially from the axis of the elongate (1).
FIG. 3 depicts another catheter according to the present invention with ablation elements (4) at their stretched position.
FIG. 4-5 depict catheters according to the present invention with ablation elements (4) expanded radially from the axis of the elongate (1).
FIG. 6 depicts the device according to the present invention.

### DETAILED DESCRIPTION OF THE INVENTION

In order to better understand the invention, the following definitions are provided: As used herein the term "subject" refers to any animal, such as a mammal like livestock, pets, and preferably a human. Specific examples of "subject" include, but are not limited, to individuals requiring medical assistance, and in particular, requiring tumor ablation treatment.

As used herein the terms "ablation" or "to ablate" and like terms, refer to what is generally known as tissue destruction or cutting procedures. Ablation is often used in treating various medical conditions including removing cancerous tissue or at least a portion of cancerous tissue.

As used herein the term "elongate" refer to what is generally known as shaft-like structure such as needle. Optionally, the elongate has lumen, i.e. it is hollow. The elongate of the present invention can be rigid or flexible. It can be made from any desired material, size and diameter. Optionally, the elongate has tubular shape. Optionally, the elongate is hallow and has tubular shape.

The terms "distal" and "proximal" as used herein refer to the relative position of the tissue and catheter. The "distal" end of the catheter is direction toward the end or at the end of the catheter closest to the tissue to be ablated. The "proximal" end of the catheter is direction toward the end or at the end of the catheter farthest from the tissue to be ablated.

As used herein the term "longitudinal" refers to a direction from the distal end to the proximal end of the elongate or vice versa.

As used herein the term "cut" or "cuts" refers to the penetration through the sliding element or shearing through the sliding element, e.g. with scissors, knife, blade, or sharpened punch. The term "cut" refers to slits where a cut through the sliding element is made. The slits or cuts can be straight or curved or wavy.

As used herein the term "cavity" refers to a hole. The hole can be round, square, rectangular, or irregular in shape. The elongate of the present invention can have a single or multiple cavities on its surface.

As used herein the term "disposed" refers to the uniform or non-uniform distribution or placement of the sliding element on the surface of the elongate.

As used herein the term "ablation element" refers to a functional element that ablates tissue such as blade. The "ablation element" can be any functional element that is designed to or capable of ablating tissue.

As used herein the term "cauterizing element" refers to an element that is designed or capable of cauterizing tissue.

As used herein, the terms "sliding", "moving", "motion" and the like have the similar meaning as generally known and are interchangeable.

The present invention provides catheters and devices that allow a surgeon to manipulate surgical target tissue as it is ablated, and optionally to remove and cauterize the target tissue with accuracy and in an efficient manner. Furthermore, the size of a surgical opening within the subject need only accommodate one device or catheter to perform said functions. In addition, the device and catheter of present invention allows a surgeon to perform tissue ablation, removal, and/or cauterization with the same device, thereby reducing the need to remove surgical devices from the subject and insert another device, thereby reducing the risk of infection and damage to surrounding tissues.

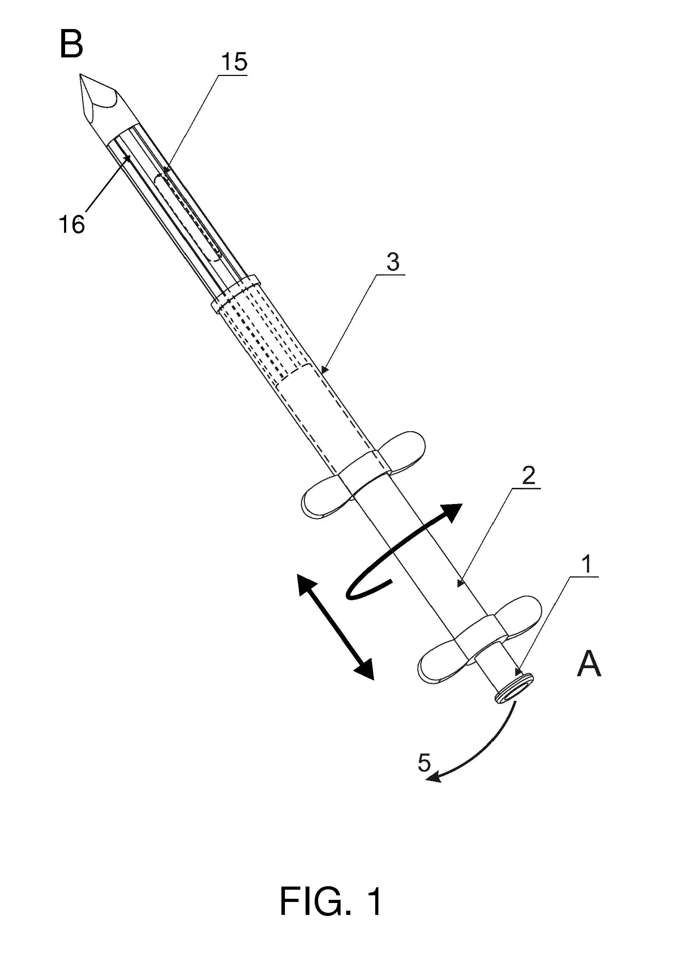

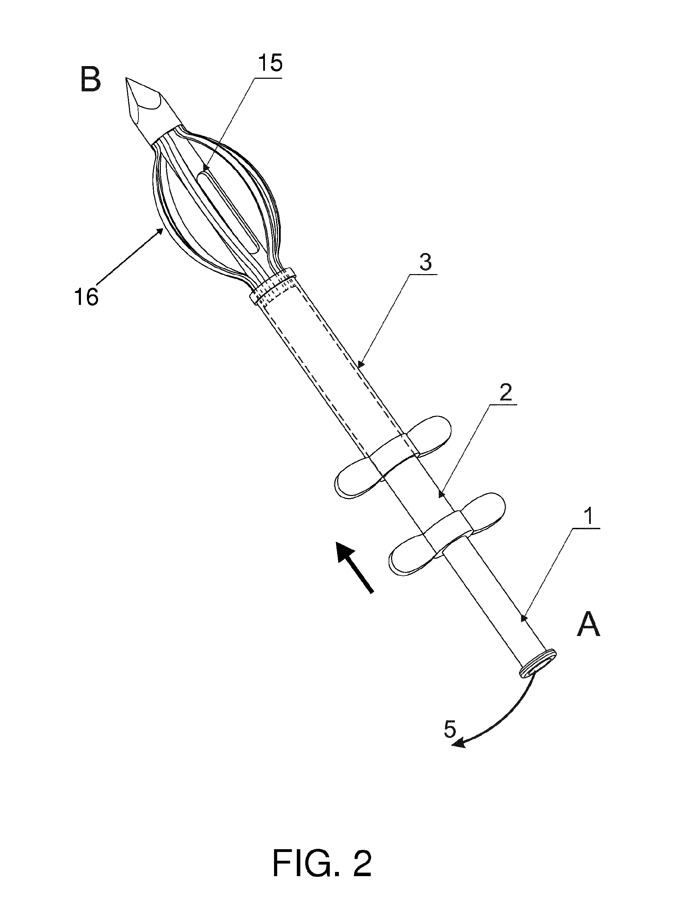

In one aspect of the invention, a catheter is provided. Referring to the drawings in the detail, FIG. 1 illustrates the catheter of the present invention being designed to ablate a biological tissue. Said catheter comprising (a) an elongate (1) having a lumen and a surface extending from a proximal end (A) to a distal end (B), wherein said elongate (1) comprising a cavity or hole (15) at the distal end (B); and (b) an sliding element (2) disposed on the surface of the elongate (1) being designed to slide in longitudinal direction of the elongate and to rotate relative to the elongate, wherein the sliding element comprising a plurality of cuts (16) at the distal end (B) of the elongate (1) and being designed to expand radially from the axis of the sliding element when said sliding element slides in the distal direction of the elongate, thereby ablating the biological tissue. For example, as illustrated in FIG. 2, the sliding element (2) is pushed in the distal direction (B) of the elongate causing the cuts (16) to expand radially. As shown in FIG. 2, the sliding element (2) being designed to be fixed at the distal end (B) of the elongate. The sliding element (2) can be any flexible material, for example, Nitinol. Once the sliding element (2) is pulled back, i.e. pulled in the proximal direction (A), the flexible cuts are returned to the stretched position.

Further, the catheter of the present invention may comprise a trocar (3) disposed on the surface of the sliding element as depicted in FIG 1 and FIG 2.

Optionally, the elongate (1) of the catheter according to any of the preceding embodiments being designed to connect to a vacuum source (5) as depicted in FIG 1 and 2, said vacuum source being designed to create a vacuum within the lumen of the elongate, thereby extracting the ablated biological tissue through the cavity (15).

The catheters of the present invention can comprise a single or multiple cuts. Optionally, the catheter of the present invention comprises 1, 2, 3, 4, 5, or 6 cuts.

In further embodiment, the cut (16) of the present invention being designed to comprise a cauterizing element, for example, where the surface of the cut (16) being designed to comprise a cauterizing element, a coating comprising a pharmaceutical agent. The cut (16) being designed to cauterize the biological tissue, for example, to prevent or stop the bleeding.

Optionally, the cauterizing element being designed to be electrically conductive and deliver electrical energy to the tissue. Optionally, the cut (16) of the present invention being designed to include an electrically insulated coating that can be connected to an electrical source or electrically conductive wire thereby cauterizing the tissue. Optionally, the cut (16) being designed to comprise chemicals to be delivered to the target tissue and to cauterize it. For example, the chemicals can be silver nitrate, phenol, and liquid nitrogen.

In further embodiment, the cut of the present invention being designed to comprise a coating of a pharmaceutical agent to be delivered to the target tissue.

The cut can have a coating containing a pharmaceutical agent, chemical cauterizing element, or electrical conductive element, or combinations thereof.

In further embodiment, The elongate (1) as depicted in Fig. 1 and 2 is being designed to connect to a vacuum source (5), said vacuum source being designed to create a vacuum within the lumen of the elongate, thereby extracting the ablated biological tissue through the cavity or hole made on the surface of the elongate.

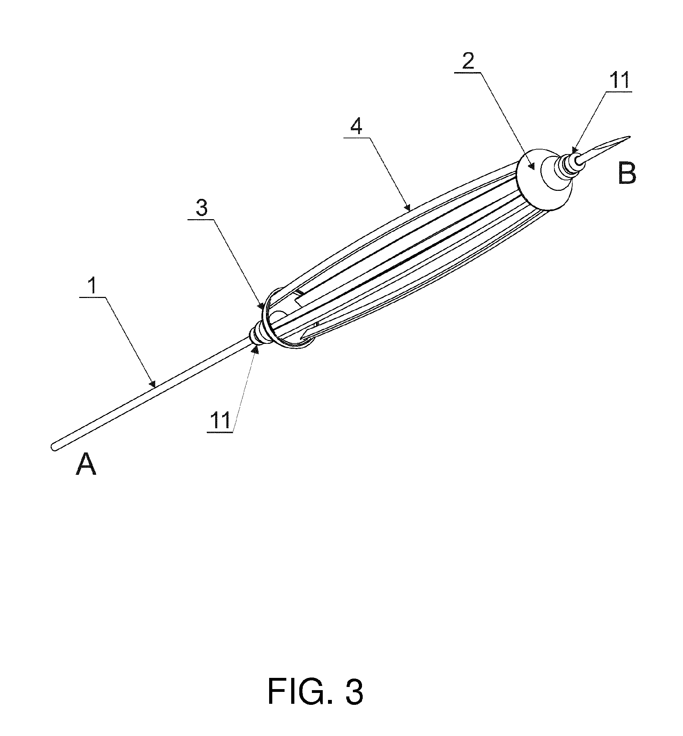

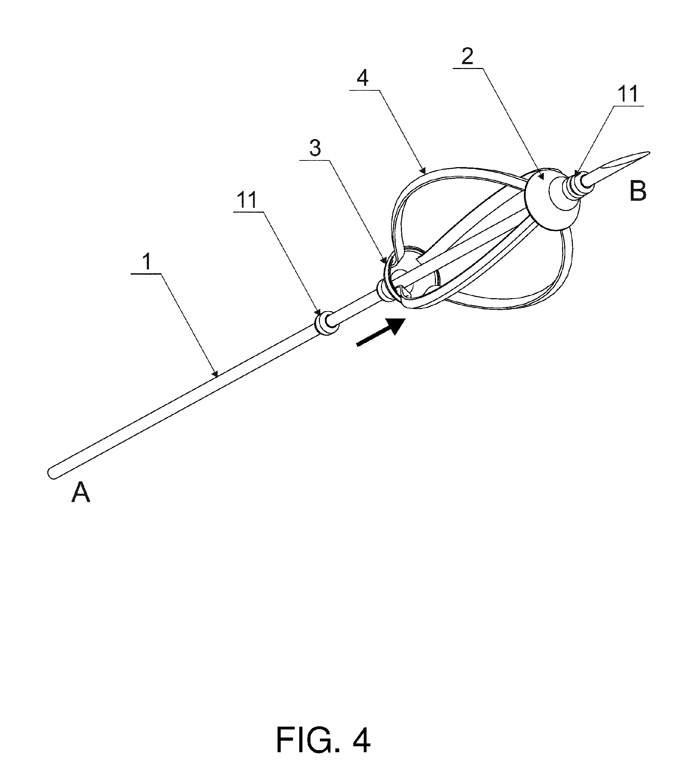

In another aspect of the invention, another catheter is provided. Referring to the drawings in detail, FIG. 3 illustrates the catheter of the present invention being designed to ablate a biological tissue from a subject comprising (a) an elongate (1) having a proximal end (A) and a distal end (B), (b) a first sliding element (2) located at the distal end of the elongate (1) being designed to slide in longitudinal direction of the elongate (1); (c) a second sliding element (3) located at the proximal end of the elongate (1) being designed to slide in longitudinal direction of the elongate (1); and (d) a plurality of flexible ablation elements (4) being designed to connect to the first (2) and the second (3) sliding elements and the ablation elements (4) being designed to expand radially from the axis of the elongate (1) when the first (2) and/or the second (3) sliding element slide in longitudinal direction of the elongate (1) as demonstrated in FIG. 3 and 4.

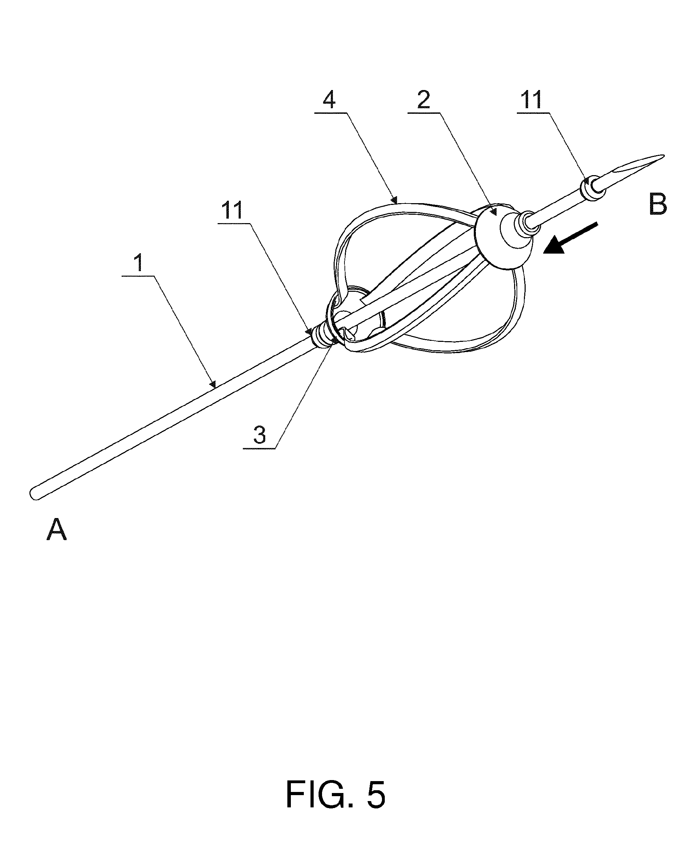

For example, as depicted in FIG 4, the sliding element (3) is pushed in longitudinal direction of the elongate (1) toward the distal end of the elongate (1), thereby causing the flexible ablation elements (4) to expand radially from the axis of the elongate and upon rotation creating a volume within the biological tissue (12). FIG 5 is another example wherein the sliding element (2) is pulled in longitudinal direction of the elongate (1) toward the proximal end of the elongate (1), thereby causing the flexible ablation elements (4) to expand radially from the axis of the elongate.

The sliding elements (2) and (3) of the present invention can move independently in longitudinal direction of the elongate. For example, the sliding elements (2) and (3) can simultaneously be pushed and/or pulled. The sliding elements (2) and (3) can have different size, shape, and made of different materials.

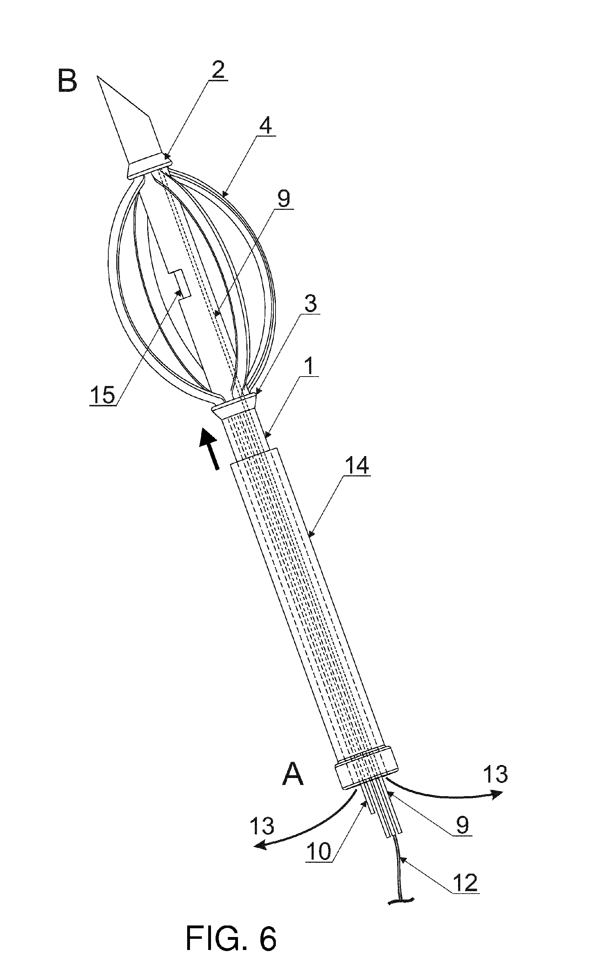

In further embodiment, the first (2) and/or the second (3) sliding element of the present invention being designed to connect to at least one controller, for example, (9) and/or (10) as illustrated in FIG. 6, wherein the at least one controller being designed to push and/or pull the first (2) and/or the second (3) sliding element in longitudinal direction of the elongate (1). For example, as depicted in FIG. 6, the sliding element (2) being designed to connect to controller (9). The controller (9) pulls the sliding element (2) in longitudinal direction of elongate (1) towards the proximal end of elongate (1). Further example, the sliding element (3) being designed to connect to controller (10). The controller (10) pushes the sliding element (3) in longitudinal direction of elongate (1) towards the distal end of elongate (1).

The controller can be a rope, wire, or trocar or any element that is able to push and/or pull the sliding elements such as a remote control. One controller or more than one controller can control the movement of the sliding elements (2) and (3). The controller can be designed to connect to the sliding elements through the lumen of the elongate as depicted in Fig. 6 or can connect to the sliding element from outside of the structure of the elongate. Optionally, the at least one controller wirelessly pushes and/or pulls the sliding elements. This means, in a further embodiment, that the sliding elements of the present invention being designed to slide in longitudinal direction of the elongate wirelessly.

In further embodiment, the first and/or the second sliding element of the present invention being designed to comprise a locking system to prevent motion of the first and/or the second sliding element. Optionally, the locking system is part of structure of the sliding elements (2) and/or (3). The locking system locks the sliding elements on the elongate preventing their motion.

As Illustrated in FIG. 3-5, in further embodiment, the elongate (1) of the present invention comprising stopping elements (11) to prevent motion of the first and/or the second sliding element in longitudinal direction of the elongate. The elongate (1) can have one stopping element or multiple stopping elements as part of its structure. Optionally, the stopping elements (11) can be placed at the any desired location on the axis of the elongate (1). Optionally, they are adjustable. Optionally, they are removable from the elongate (1).

The catheters of the present invention can comprise multiple ablation elements. Optionally, the catheter of the present invention comprises 2, 3, 4, 5, or 6 cuts or ablation elements.

In further embodiment, the ablation element (4) of the present invention being designed to comprise a cauterizing element. For example. the surface of the ablation element (4) comprises a cauterizing element, a coating comprising a pharmaceutical agent and an edge of the ablation element. The cauterizing elements designed to cauterize the biological tissue, for example, to prevent or stop the bleeding.

Optionally, the cauterizing element being designed to be electrically conductive and deliver electrical energy to the tissue. Optionally, the ablation element of the present invention being designed to include an electrically insulated coating that can be connected to an electrical source or electrically conductive wire thereby cauterizing the tissue. Optionally, the cauterizing element being designed to comprise chemicals to be delivered to the target tissue and to cauterize it. For example, the chemicals can be silver nitrate, phenol, and liquid nitrogen.

In further embodiment, the ablation element of the present invention being designed to comprise a coating of a pharmaceutical agent to be delivered to the target tissue.

The ablation element of present invention can have a coating containing a pharmaceutical agent, chemical cauterizing element, or electrical conductive element, or combinations thereof.

In another aspect of the invention, a device being designed to ablate the biological tissue from a subject is provided. Referring to FIG. 6, said device comprising (a) a housing (7) having a lumen, for example, a trocar; (b) a catheter comprising (i) an elongate (1) having a proximal end and a distal end (B), (ii) a first sliding element (2) located at the distal end of the elongate being designed to slide in longitudinal direction of the elongate; (iii) a second sliding element (3) located at the proximal end of the elongate being designed to slide in longitudinal direction of the elongate; (iv) a plurality of flexible ablation elements (4) being designed to connect to the first and the second sliding elements and the ablation elements being designed to expand radially from the axis of the elongate when the first and/or the second sliding element slide in longitudinal direction of the elongate; wherein the catheter is disposed within the lumen and said elongate is both movable and rotatable relative to the hosing, thereby moving and rotating the sliding and ablation elements; and (c) at least one controller, for example, (9) and (10) being designed to move the first and/or the second sliding element in longitudinal direction of the elongate and to rotate the elongate, thereby ablating the biological tissue.

In further embodiment, the device of the present invention further comprising a vacuum source (8) designed to create a vacuum within the housing (7), thereby extracting the ablated biological tissue.

In further embodiment, the device of the present invention comprising a source of electrical energy (11) eclectically coupled to the cauterizing elements.

The catheter, sliding elements, ablation elements of the device are according to any of the preceding embodiments and can be combined with the device of the present invention. The catheter of the device can be disposable or replaceable. Optionally, the housing and the catheter are one-piece, i.e. the components of the device are not replaceable and/or detachable, which optionally makes the device disposable.

In further embodiment, the catheters and the devices of the present invention according to any of the preceding embodiments being designed to be monitored via Real-time imaging to assist in placement of the catheter and device and in ablating and removing the biological tissue. Real-time imaging technologies include, but not limited to, X-ray radiography, magnetic resonance imaging, medical ultrasonography or ultrasound, medical photography, nuclear medicine functional imaging, positron emission tomography, radiology, and CT Scan.

Additional embodiments provide methods of ablating a biological tissue from a subject.

### OTHER EMBODIMENTS

The detailed description set-forth above is provided to aid those skilled in the art in practicing the present invention. However, the invention described and claimed herein is not to be limited in scope by the specific embodiments herein disclosed because these embodiments are intended as illustration of several aspects of the invention. The embodiments set-forth above can be performed and combined with other disclosed embodiments according to the invention. Any equivalent embodiments are intended to be within the scope of this invention. Indeed, various modifications of the invention in addition to those shown and described herein will become apparent to those skilled in the art from the foregoing description which do not depart from the spirit or scope of the present inventive discovery. Such modifications are also intended to fall within the scope of the appended claims.

The phrase "and/or" as used herein in the specification and in the claims, should be understood to mean "either or both". As a non-limiting example, a reference to " the monoblock and/or modular" can refer, in one embodiment, to the monoblock component only (optionally including elements other than modular); in another embodiment, to modular component only (optionally including elements other than the monoblock component); in yet another embodiment, to both the monoblock and modular components (optionally including other elements); etc.

## Claims

1. A catheter being designed to ablate a biological tissue from a subject, comprising:
a. An elongate having a lumen and a surface extending from a proximal end to a distal end, wherein said surface of the elongate comprising a cavity at the distal end; and
b. An sliding element disposed on the surface of the elongate being designed to slide in longitudinal direction of the elongate and to rotate relative to the elongate, wherein the sliding element comprising a plurality of cuts at the distal end of the elongate and being designed to expand radially from the axis of the sliding element when said sliding element slides in the distal direction of the elongate, thereby ablating the biological tissue.

2. The catheter of claim 1, said catheter further comprising a trocar disposed on the sliding element.

3. The catheter according to claim 1, wherein said elongate being designed to connect to a vacuum source, said vacuum source being designed to create a vacuum within the lumen of the elongate, thereby extracting the ablated biological tissue through the cavity.

4. The catheter according to claim 1, wherein the cut being designed to comprise a cauterizing element.

5. The catheter according to claim 4, wherein the cauterizing element being designed to be electrically conductive.

6. The catheter according to claim 4, wherein the cauterizing element being designed to release cauterizing chemicals.

7. The catheter according to claim 1, wherein the cut being designed to comprise a coating containing a pharmaceutical agent.

8. The catheter according to claim 1, wherein said catheter being designed to be monitored via Real-time imaging to assist in placement of the catheter and device and in ablating and removing the biological tissue.
